# EUROPEAN PATENT SPECIFICATION

(11) **EP 2 566 569 B1**
(45) Date of publication and mention of the grant of the patent: **18.07.2018**
(21) Application number: 10851861.4
(22) Date of filing: 21.05.2010
(51) Int. Cl.: A61J 1/10, A61M 5/162, A61M 39/04, F16L 37/53, A61M 39/02, A61J 1/14, A61M 39/10

(54) **CONNECTOR, FLUID CONTAINER**
ANSCHLUSS, FLÜSSIGKEITSBEHÄLTER
CONNECTEUR, RÉCIPIENT POUR FLUIDES

(43) Date of publication of application: 13.03.2013
(73) Proprietor: Carmel Pharma AB, 402 28 Göteborg (SE)
(72) Inventor: ROSENQUIST, Tobias, S-428 34 Kållered (SE); LINDSTRÖM, Johanna, 43642 Askim (SE); CEDERSHIÖLD, Alexander, 41257 Göteborg (SE); LEFFLER, Jonas, 41257 Göteborg (SE)
(74) Representative: dompatent von Kreisler Selting Werner - Partnerschaft von Patent- und Rechtsanwälten mbB
(86) International application number: PCT/SE2010/050555
(87) International publication number: WO 2011/145991

(56) References cited:
- WO-A1-00/53966
- WO-A1-2008/009646
- WO-A1-2010/007422
- US-A- 5 222 486
- US-A1- 2007 026 703
- US-A1- 2008 090 445
- US-A1- 2009 312 717

## Description

### TECHNICAL FIELD

The present invention concerns a connector for enabling fluid transfer between a first fluid container and a second fluid container, such as a syringe or needle protector. The present invention also concerns a fluid container comprising at least a part of such a connector.

### BACKGROUND OF THE INVENTION

Many medical connectors comprise a first component having a female luer-lock element that is arranged to be rigidly joined to a corresponding male luer-lock element of a second connector component that is attached to a medical line for example. The male luer lock element can thus be freely screwed into and unscrewed from the female luer-lock element. However, once the male luer-lock element has been screwed into the female luer-lock element of the connector, there is a risk that the connector components may be accidentally or inadvertently unscrewed, which could lead to the disconnection of the fluid line. This may entail a serious contamination risk for a patient and or any other person in the vicinity of the disconnected medical connector. Such a disconnection risk must especially be avoided when administering toxic fluid, such as cytostatic agents.

Some medical connectors are arranged so as to be non-disconnectable once they have been connected to a medical line for example. A disadvantage with such a connector is that it may induce an undesirable twist in the medical line when a second fluid container, such as a syringe or a needle protector, is secured to the connector in order to enable fluid transfer between the medical line and the second fluid container.

US 2009/0312717 A1 describes a medical delivery system with a rotatable coding element. The medical delivery system comprises a container, a dosing element and a rotatable element, which is adapted to engage the container and the dosing element. After engagement, a rotatable connection of the components is achieved.

Additionally, WO 2010/007422 A1 discloses the features of the preamble of claim 1.

### SUMMARY OF THE INVENTION

An object of the invention is to overcome or ameliorate at least one of the disadvantages of the prior art, or to provide a useful alternative. A further object of the present invention is to provide a safe and reliable connector for enabling fluid between a first fluid container and a second fluid container, such as a syringe or needle protector.

At least one of these objects is achieved by a connector that comprises a first component that is arranged to be connected to a first fluid container and a second component that is arranged to be connected to a second fluid container. The first component is arranged to
be non-rotatably connected to the second component, or a part of the second component, and to a first fluid container before a second fluid container is connected to the second component and/or before a first fluid container is connected to the first component. The connector comprises a locking and rotation-enabling element that is arranged to be activated once a second fluid container has been connected to the second component and/or once a first fluid container has been connected to the first element, which locking and rotation-enabling element enables the first component and the second component to rotate freely with respect to one another, i.e. rotate freely in one direction or a plurality of directions without hindrance, or to rotate freely in one direction or a plurality of directions for at least part of one revolution or any number of revolutions before it is prevented from rotating further. Once the locking and rotation-enabling element has been activated, it will not be possible to accidentally or inadvertently disconnect the first component from the second component, whereby the locking of the first and the second components is irreversible. According to an alternative embodiment of the present invention the first component is arranged to be disconnectable from the second component once the locking and rotation-enabling element has been activated, whereby the locking of the first and the second components is reversible.

The expressions "first fluid container" and "second fluid container" as used in this document are intended to mean any vessel that can at least temporarily contain a fluid, such as a vial, a medical line, a tube or an infusion fluid container, such as an infusion bottle or an infusion bag, a syringe or a needle protector device. The expression "a locking and rotation-enabling element that is arranged to be activated once a second fluid container has been connected to the second component and/or once a first fluid container has been connected to the first component" is intended to mean that the locking and rotation-enabling element is arranged to be activated either when one or both of the vessels that are to be connected to the connector. For example, if the locking and rotation-enabling element is arranged to be activated when it has been linearly, nonlinearly or rotationally displaced a certain distance in for example the longitudinal direction of the connector, the connector may be arranged so that the connection of one or both of said vessels and/or the manual activation of a user may be required in order to displace the locking and rotation-enabling element the required distance.

It should be noted that the expression "the locking and rotation-enabling element" is intended to mean a single component or a plurality of components that are arranged to lock and to enable the rotation of the first component with respect to the second component.

The first and the second components of the connector according to the present invention may therefore be connected together so that they will not rotate with respect to each other until a second fluid container and/or a first fluid container is connected to the connector. On connection of a second fluid container and/or a first fluid container, the locking and rotation-enabling element will be activated and the first and the second components will then be able to rotate freely with respect to one another. The free rotation will clearly indicate to a user that a non-disconnectable connection has been made, i.e. that the first and the second components can not be separated without breaking the connector, using excessive force, or mis-using the connector. Furthermore, any undesired twists in the first fluid container or second fluid container may also be remedied by rotating the first and/or the second component of the connector.

According to an embodiment of the invention one of the first or second component comprises at least one protrusion that is arranged to become located in at least one corresponding cavity in the other of the first or second component or in the locking and rotation-enabling element, in order to connect the first component to the second component (or a part thereof) in a non-rotatable manner. The locking and rotation-enabling element is arranged to force the at least one protrusion out of the at least one corresponding cavity, whereby the first component and the second component are enabled to rotate freely with respect to one another when the at least one protrusion is forced out of the at least one corresponding cavity.

According to another embodiment of the invention the locking and rotation-enabling element is arranged to force the at least one protrusion out of the at least one corresponding cavity and into at least one slot, whereby the first component and the second component are enabled to rotate freely with respect to one another when the at least one protrusion is located in the at least one slot.

According to a further embodiment of the invention the locking and rotation-enabling element is arranged to be automatically activated when the second fluid container and/or first fluid container is/are connected to the connector. According to an embodiment of the invention the locking and rotation-enabling element is arranged to be automatically activated when it is directly or indirectly displaced by the second fluid container and/or the first fluid container when the second fluid container and/or the first fluid container is/are subsequently being connected to the connector, whereby the displacement of the locking and rotation-enabling element forces the at least one protrusion out of the at least one cavity.

Alternatively, in the connector according to the present invention the locking and rotation-enabling element is arranged to be manually activated by a user after the second fluid container and/or first fluid container has/have been connected to the connector. According to an embodiment of the invention the locking and rotation-enabling element is arranged to be manually activated by a user that directly or indirectly causes it to be displaced after the second fluid container and/or first fluid container has/have been connected to the connector, whereby the displacement of the locking and rotation-enabling element forces the at least one protrusion out of the at least one cavity.

According to another embodiment of the invention the connector comprises a snap fit mechanism to connect the first component to the second component. A snap fit mechanism is a self-locking joint whose mating parts exert a cam action, flexing until one part slips past a raised lip on the other part, preventing their separation.

According to a further embodiment of the invention the first component is arranged to be screwed into the second component. Alternatively, the first component is arranged to be slid into the second component.

According to an embodiment of the invention the first component and/or the second component comprises a membrane. The membrane of a second fluid container or a first fluid container may be pressed against the membrane of the connector component to form a double membrane and a piercing member, such as a needle, may then penetrate the double membrane in order to achieve leak free fluid transfer.

According to another embodiment of the present invention the connector comprises sealing means, such an o-ring or gasket, to ensure that a fluid-tight, leak-free connection is made between the first fluid container and the second fluid container.

The present invention also concerns a fluid container that comprises an integrally formed first component of a connector according to any of the embodiments of the invention. The connector according to the present invention may however be arranged to be connected to any fluid container.

### BRIEF DESCRIPTION OF THE DRAWINGS

The present invention will hereinafter be further explained by means of non-limiting examples with reference to the appended schematic figures where;
- Figure 1: shows an exploded view of a connector according to an embodiment of the invention,
- Figure 2: depicts a locking and rotation-enabling element of a connector according to an embodiment of the invention,
- Figure 3: shows a first component of a connector according to an embodiment of the invention,
- Figures 4: shows a first component connected to a locking and rotation-enabling element in a non-rotatable manner,
- Figures 5 & 6: show a first component connected to a locking and rotation-enabling element in a manner that enables the first component to rotate freely with respect to the locking and rotation-enabling element,
- Figure 7: shows a connector according to an embodiment of the invention in which a first and second component are connected in a non-rotatable manner,
- Figure 8: shows a connector according to an embodiment of the invention in which a first and second component are connected in a rotatable manner,
- Figures 9 & 10: show a second component of connector according to different embodiments of the invention, and
- Figures 11 & 12: show a fluid container comprising at least a component of a connector according to the present invention.

It should be noted that the drawings have not been drawn to scale and that the dimensions of certain features have been exaggerated for the sake of clarity.

### DETAILED DESCRIPTION OF EMBODIMENTS

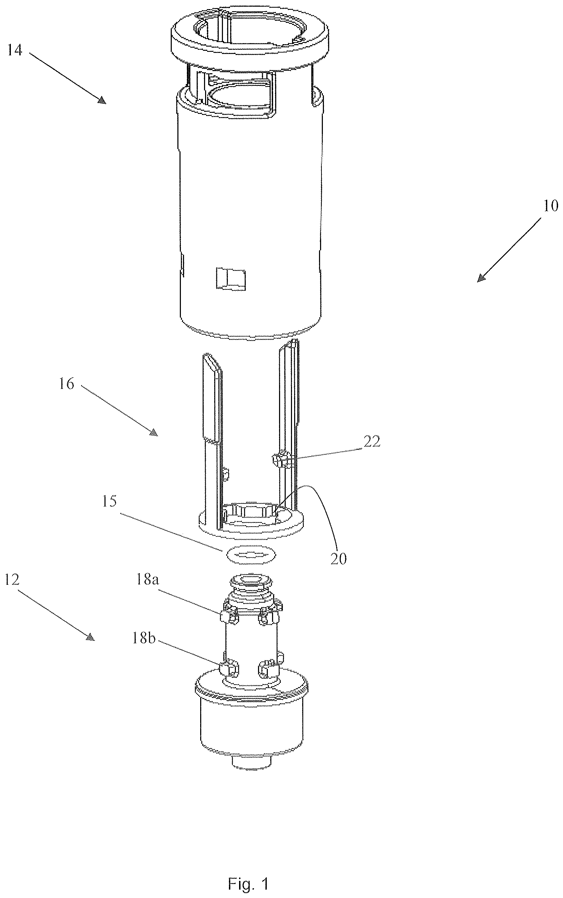

Figure 1 shows an exploded view of a connector 10 for enabling fluid transfer between a first fluid container and a second fluid container. The connector 10 comprises a first component 12 that is arranged to be connected to a first fluid container and a second component 14 that is arranged to be connected to a second fluid container. The first and the second component 12 and 14 in the illustrated embodiment are tubular and comprise at least one fluid channel (not shown) through which fluid may flow through the connector. The connector 10 comprises an o-ring 15 to ensure that a fluid-tight, leak-free connection is made between the first fluid container and the second fluid container.

The first component 12 is arranged to be non-rotatably connected to the second component 14 and to a first fluid container before a second fluid container is connected to the second component 14. The connector 10 comprises a locking and rotation-enabling element 16 that is arranged to be activated once a second fluid container has subsequently been connected to the second component 14. In the illustrated embodiment the locking and rotation-enabling element 16 constitutes part of the second component 14 and is non-rotatable, and slidably displaceable with respect to the second component 14. The locking and rotation-enabling element 16 enables the first component 12 and the second component 14 to rotate freely with respect to one another when it has been directly or indirectly activated (automatically or manually).

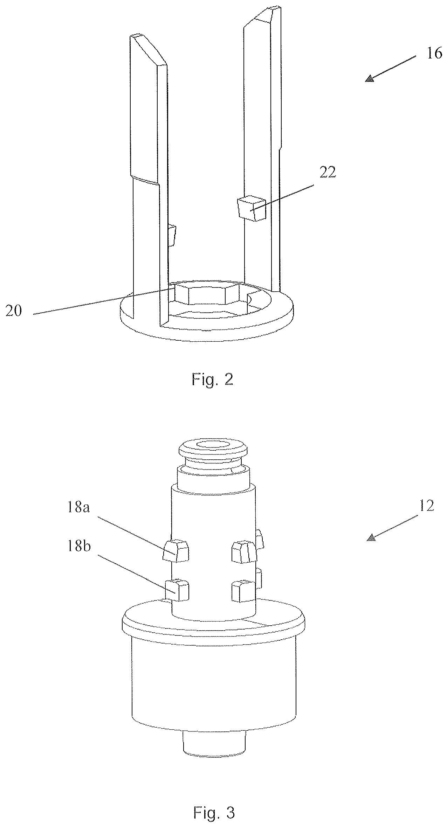

With reference to figures 1-3 the first component 12 comprises two levels of protrusions 18a and 18b. The lower level of protrusions 18b is arranged to become located in corresponding cavities 20 (whereby the turning force applied to the first component 12 will be transferred to the locking and rotation-enabling element 16) in the locking and rotation-enabling element 16 of the second component 14 in order to connect the first component 12 to the second component 14 in a non-rotatable manner. The upper level of protrusions 18a is arranged to become located adjacent to a row of protrusions 22 (shown in figure 1) on the locking and rotation-enabling element 16 in a snap fit manner for example.

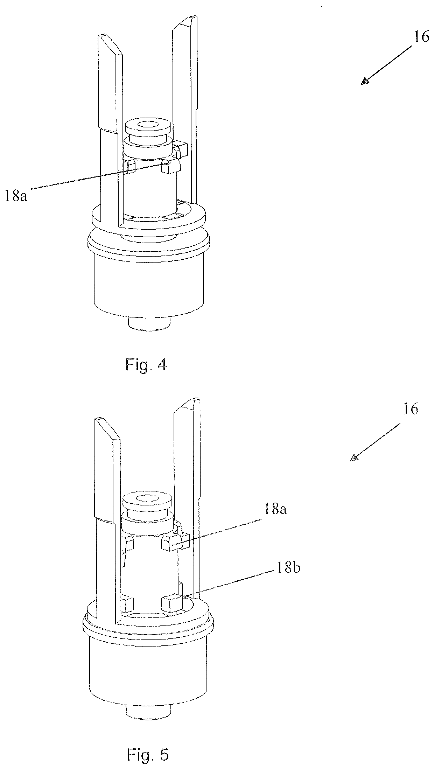

Figure 4 shows the first component 12 connected to the locking and rotation-enabling element 16, whereby the lower level of protrusions 18b is located in cavities 20 in the locking and rotation-enabling element 16.

When a second fluid container is connected to the second component 14 the locking and rotation-enabling element 16 will be slidably displaced downwards to the position shown in figure 5 and will force the lower level of protrusions 18b out of the cavities 20, whereby the first component 12 and locking and rotation-enabling element 16 (and consequently the second component 14) will then be able to rotate freely with respect to one another.

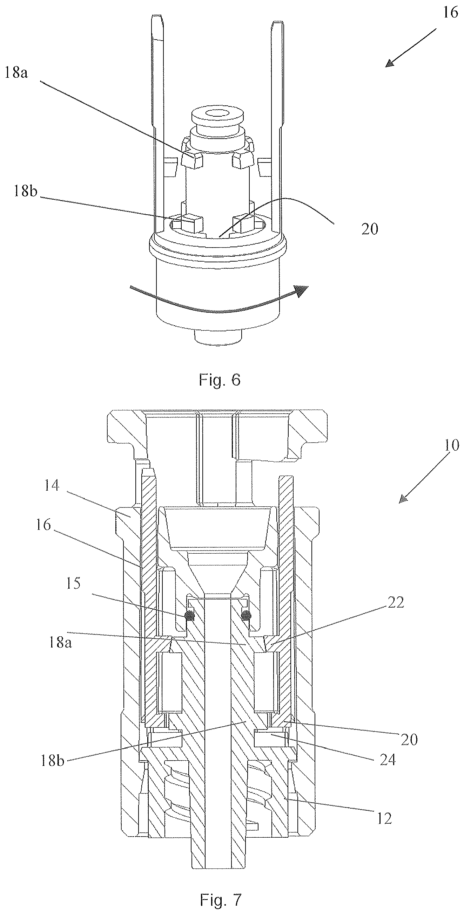

Figure 6 shows the first component 12 in a position in which it has been rotated in the direction of the arrow in figure 6, whereby the cavities 20 in the locking and rotation-enabling element 16 are located slightly to the right of the lower level of protrusions 18b. It should be noted that the connector 10 may be arranged to also enable rotation in the direction opposite to the direction of the arrow in figure 6. According to an embodiment of the invention the connector 10 may comprise means to prevent the first component 12 and the second component 14 from returning to their unlocked position in which they cannot rotate with respect to one another. For example, in the embodiment illustrated in figure 6, the connector 10 may be provided with a spring-loaded mechanism to prevent the protrusions 18b from being displaced back into the cavities 20.

Figure 7 shows a cross section of the first component 12 and the second component 14 that includes the locking and rotation-enabling element 16, when the first component 12 and the second component 14 are connected in a non-rotatable manner with respect to one another, i.e. when the lower level of protrusions 18b is located in the cavities 20 in the locking and rotation-enabling element 16. The upper level of protrusions 18a rest against the protrusions 22 of the locking and rotation-enabling element 16. The connector shown in figure 7 comprises a slot 24 just below the lower level of protrusions 18b.

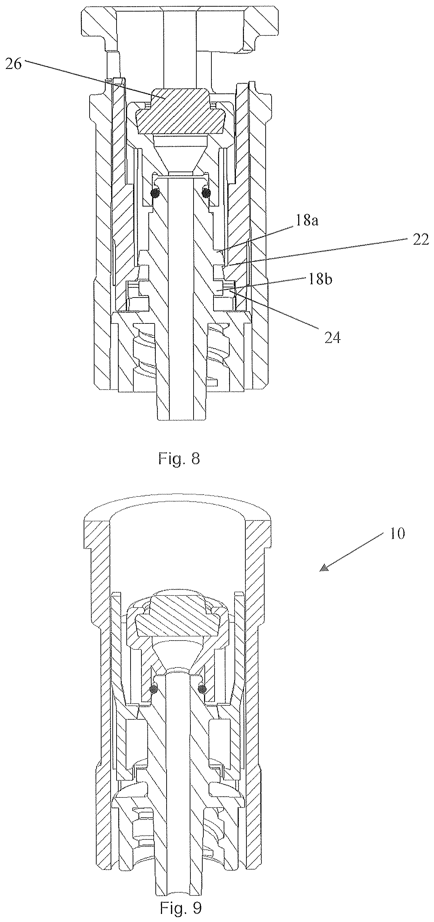

When the locking and rotation-enabling element 16 is activated, it will be displaced downwards in figure 7 to the position shown in figure 8. In this position the lower level of protrusions 18b will become located in the slot 24 and the upper level of protrusions 18a will no longer rest against the protrusions 22 of the locking and rotation-enabling element 16. The first component 12 will therefore be able to rotate freely with respect to the locking and rotation-enabling element 16 and consequently with respect to the second component 14. Furthermore, since the protrusions 22 of the locking and rotation-enabling element 16 are now located between the upper and lower levels of protrusions 18a and 18b, the first component 12 will remain permanently connected to the second component 14 and will preferably not be separable therefrom without breaking the connector 10, using excessive force or mis-using the connector 10. In the illustrated embodiment the second component 14 comprises a membrane 26 that may be placed in tight apposition against the membrane of a second fluid container, such as a needle protector, to ensure leak-free fluid transfer between the second fluid container and the second component 14.

In the connector embodiment shown in figures 1-8, the locking and rotation-enabling element 16 is arranged to be directly and automatically activated when a second fluid container is connected to the second component 14. The connection of a second fluid container to the second component 14 namely causes the locking and rotation-enabling element 16 to be slidably displaced in a longitudinal direction (downwards in figures 1-8) to enable the first component 12 to be freely rotatable with respect to the second component 14. Alternatively, such a slideable displacement may be achieved by a user manually activating a lever on the side of the connector for example once a second fluid container has been connected to the second component 14, which lever activates the locking and rotation-enabling element 16.

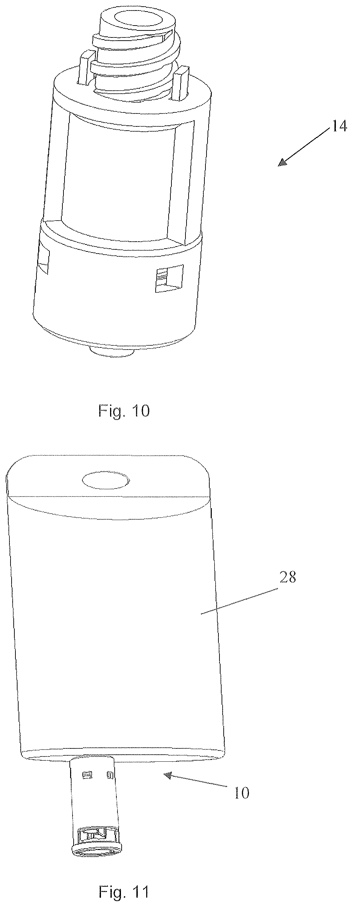

Figure 9 shows a connector 10 comprising a snap fit mechanism 17. Figure 10 shows that the second component 14 may be arranged to be screwed into a second fluid container. It should be noted that the first component 12 may also be arranged to be slid or screwed into a first fluid container. Furthermore, the first component 12 may be arranged to be slid or screwed into the second component 14 and the locking and rotation-enabling element 16 may be arranged to be displaced in a linear, non-linear or rotatable manner.

Figure 11 shows a first fluid container 28, namely an infusion bag, comprising an integrally formed connector 10 according to the present invention. Such a connector 10 may alternatively be arranged to be temporarily or permanently connected to the infusion port or the injection port of a standard infusion bag.

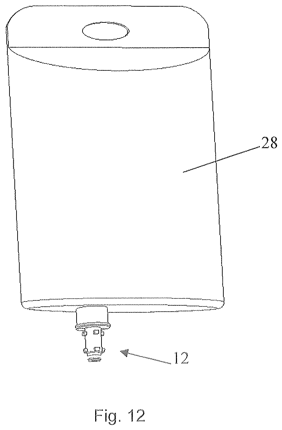

Figure 12 shows a first fluid container 28, namely an infusion bag, comprising an integrally formed first component 12 of a connector 10 according to the present invention. Such a first component 12 may alternatively be arranged to be temporarily or permanently connected to the infusion port or the injection port of a standard infusion bag.

Further modifications of the invention within the scope of the claims would be apparent to a skilled person. For example, the first component 12 and the second component 14 may be connected between any two vessels between which fluid transfer is desired. The first component 12 may be arranged to be connected to the second component 14 (or a part thereof such as a locking and rotation-enabling element 16) in any suitable manner. There are many ways of designing and arranging a locking and rotation-enabling element 16 to enable free rotation between the first component 12 and the second component 14 of the connector 10 once a second fluid container and/or a medical container has/have been connected to the connector. The design and arrangement shown in the figures merely provides one such example in order to illustrate the principle of the present invention. Furthermore, it should be noted that although it is easier to manufacture a locking and rotation-enabling element 16 that is directly activated by the connection of a second fluid container and/or a first fluid container to the connector 10, the connector may be provided with an additional mechanism to indirectly automatically or manually activate the locking and rotation-enabling element 16.

## Claims

1. Connector (10) for enabling fluid transfer between a first fluid container (28) and a second fluid container, which connector (10) comprises a first component (12) that is arranged to be connected to a first fluid container (28) and a second component (14) that is arranged to be connected to a second fluid container, whereby said first component (12) is arranged to be non-rotatably connected to said second component (14), or a part of the second component (14), and to a first fluid container (28) before a second fluid container is connected to said second component (14) and/or before a first fluid container (28) is connected to the first component (12), **characterized in that** said connector (10) comprises a locking and rotation-enabling element (16) that is arranged to be activated **in that** said rotation-enabling element (16) is non-rotatably and slidably displaced relative to the second component (14) once a second fluid container has been connected to said second component (14), and/or once a first fluid container (28) has been connected to the first component (12), which activation of the locking and rotation-enabling element (16) enables the first component (12) and the second component (14) to rotate freely with respect to one another.

2. Connector (10) according to claim 1 , **characterized in that** one of said first (12) or second component (14) comprises at least one protrusion (18b) that is arranged to become located in at least one corresponding cavity (20) in the other of said first or second component (14) or in said locking and rotation-enabling element (16), in order to connect said first component (12) to said second component (14) in a non-rotatable manner, and whereby said locking and rotation-enabling element (16) is arranged to force said at least one protrusion (18b) out of said at least one corresponding cavity (20), whereby said first component (12) and said second component (14) are enabled to rotate freely with respect to one another when said at least one protrusion (18b) is forced out of said at least one corresponding cavity (20).

3. Connector (10) according to claim 2, **characterized in that** said locking and rotation-enabling element (16) is arranged to force said at least one protrusion (18b) out of said at least one corresponding cavity (20) and into at least one slot (24), whereby said first component (12) and said second component (14) are enabled to rotate freely with respect to one another when said at least one protrusion (18b) is located in said at least one slot (24).

4. Connector (10) according to any of the preceding claims, **characterized in that** said locking and rotation-enabling element (16) is arranged to be automatically activated when said second fluid container is connected to said second component (14) and/or once a first fluid container (28) has been connected to the first component (12).

5. Connector (10) according to claim 4 when dependent on claim 2 or 3, **characterized in that** said locking and rotation-enabling element (16) is arranged to be automatically activated when it is directly or indirectly displaced by said second fluid container when said second fluid container is subsequently being connected to said second component (14), whereby said displacement of said locking and rotation-enabling element (16) forces said at least one protrusion (18b) out of said at least one cavity (20) and/or when it is directly or indirectly displaced by said first fluid container (28) when said first fluid container (28) is subsequently being connected to said first component (12), whereby said displacement of said locking and rotation-enabling element (16) forces said at least one protrusion (18b) out of said at least one cavity (20).

6. Connector (10) according to any of claims 1 -3, **characterized in that** said locking and rotation-enabling element (16) is arranged to be manually activated by a user after said second fluid container has been connected to said second component (14) and/or when said first fluid container (28) has been connected to said first component (12).

7. Connector (10) according to claim 6 when dependent on claim 2 or 3, **characterized in that** said locking and rotation-enabling element (16) is arranged to be manually activated by a user that directly or indirectly causes it to be displaced after said second fluid container has been connected to said second component (14) and/or said first fluid container (28) has been connected to said first component (12), whereby said displacement of said locking and rotation-enabling element (16) forces said at least one protrusion (18b) out of said at least one cavity (20).

8. Connector (10) according to any of the preceding claims, **characterized in that** it comprises a snap fit mechanism (17) to connect said first component (12) to said second component (14) or said rotation-enabling element (16).

9. Connector (10) according to any of the preceding claims, **characterized in that** said first component (12) is arranged to be screwed into said second component (14).

10. Connector (10) according to any of claims 1-8, **characterized in that** said first component (12) is arranged to be slid into said second component (14).

11. Connector (10) according to any of the preceding claims, **characterized in that** said second component (14) and/or said first component (12) comprises a membrane.

12. Connector (10) according to any of the preceding claims, **characterized in that** it comprises sealing means (15) to ensure that a fluid-tight, leak-free connection is made between the first fluid container (28) and the second fluid container.

## Patentansprüche

1. Anschluss (10) zum Ermöglichen einer Flüssigkeitsübertragung zwischen einem ersten Flüssigkeitsbehälter (28) und einem zweiten Flüssigkeitsbehälter, wobei der Anschluss (10) eine erste Komponente (12), die derart angeordnet ist, dass sie mit einem ersten Flüssigkeitsbehälter (28) verbunden wird, und eine zweite Komponente (14) umfasst, die derart angeordnet ist, dass sie mit einem zweiten Flüssigkeitsbehälter verbunden wird, wodurch die erste Komponente (12) derart angeordnet ist, dass sie nichtdrehbar mit der zweiten Komponente (14) oder einem Teil der zweiten Komponente (14) und mit einem ersten Flüssigkeitsbehälter (28) verbunden wird, bevor ein zweiter Flüssigkeitsbehälter mit der zweiten Komponente (14) verbunden wird und/oder bevor ein erster Flüssigkeitsbehälter (28) mit der ersten Komponente (12) verbunden wird,
**dadurch gekennzeichnet, dass**
der Anschluss (10) ein Verriegelungs- und Drehermöglichungselement (16) umfasst, das derart angeordnet ist, dass es dadurch aktiviert wird, dass das Drehermöglichungselement (16) relativ zu der zweiten Komponente (14) nichtdrehbar und gleitend verschoben wird, sobald ein zweiter Flüssigkeitsbehälter mit der zweiten Komponente (14) verbunden worden ist und/oder sobald ein erster Flüssigkeitsbehälter (28) mit der ersten Komponente (12) verbunden worden ist, wobei die Aktivierung des Verriegelungs- und Drehermöglichungselements (16) die erste Komponente (12) und die zweite Komponente (14) in die Lage versetzt, sich relativ zueinander frei zu bewegen.

2. Anschluss (10) nach Anspruch 1, **dadurch gekennzeichnet, dass** eine der ersten (12) oder der zweiten Komponente (14) mindestens einen Vorsprung (18b) umfasst, der derart angeordnet ist, dass er sich in zumindest einem entsprechenden Hohlraum (20) in der anderen der ersten oder der zweiten Komponente (14) oder in dem Verriegelungs- und Drehermöglichungselement (16) befindet, um die erste Komponente (12) auf nichtdrehbare Weise mit der zweiten Komponente (14) zu verbinden, und wodurch das Verriegelungs- und Drehermöglichungselement (16) derart angeordnet ist, dass es den mindestens einen Vorsprung (18b) aus dem mindestens einen entsprechenden Hohlraum (20) drückt, wodurch die erste Komponente (12) und die zweite Komponente (14) in der Lage sind, sich relativ zueinander frei zu drehen, wenn der mindestens eine Vorsprung (18b) aus dem mindestens einen entsprechenden Hohlraum (20) gedrückt wird.

3. Anschluss (10) nach Anspruch 2, **dadurch gekennzeichnet, dass** das Verriegelungs- und Drehermöglichungselement (16) derart angeordnet ist, dass es den mindestens einen Vorsprung (18b) aus dem mindestens einen entsprechenden Hohlraum (20) und in mindestens einen Schlitz (24) drückt, wodurch die erste Komponente (12) und die zweite Komponente (14) in die Lage versetzt werden, sich relativ zueinander frei zu drehen, wenn sich der mindestens eine Vorsprung (18b) in dem mindestens einen Schlitz (24) befindet.

4. Anschluss (10) nach einem der vorhergehenden Ansprüche, **dadurch gekennzeichnet, dass** das Verriegelungs- und Drehermöglichungselement (16) derart angeordnet ist, dass es automatisch aktiviert wird, wenn der zweite Flüssigkeitsbehälter mit der zweiten Komponente (14) verbunden ist und/ oder sobald ein erster Flüssigkeitsbehälter (28) mit der ersten Komponente (12) verbunden worden ist.

5. Anschluss (10) nach Anspruch 4 bei Rückbezug auf Anspruch 2 oder 3, **dadurch gekennzeichnet, dass** das Verriegelungs- und Drehermöglichungselement (16) derart angeordnet ist, dass es automatisch aktiviert wird, wenn es direkt oder indirekt von dem zweiten Flüssigkeitsbehälter verschoben wird, wenn der zweite Flüssigkeitsbehälter anschließend mit der zweiten Komponente (14) verbunden wird, wodurch durch das Verschieben des Verriegelungs- und Drehermöglichungselements (16) der mindestens eine Vorsprung (18b) aus dem mindestens einen Hohlraum (20) gedrückt wird, und/oder wenn es direkt oder indirekt von dem ersten Flüssigkeitsbehälter (28) verschoben wird, wenn der erste Flüssigkeitsbehälter (28) anschließend mit der ersten Komponente (12) verbunden wird, wodurch durch das Verschieben des Verriegelungs- und Drehermöglichungselements (16) der mindestens eine Vorsprung (18b) aus dem mindestens einen Hohlraum (20) gedrückt wird.

6. Anschluss (10) nach einem der Ansprüche 1-3, **dadurch gekennzeichnet, dass** das Verriegelungs- und Drehermöglichungselement (16) derart angeordnet ist, dass es von einem Benutzer manuell aktiviert wird, nachdem der zweite Flüssigkeitsbehälter mit der zweiten Komponente (14) verbunden worden ist und/oder wenn der erste Flüssigkeitsbehälter (28) mit der ersten Komponente (12) verbunden worden ist.

7. Anschluss (10) nach Anspruch 6 bei Rückbezug auf Anspruch 2 oder 3, **dadurch gekennzeichnet, dass** das Verriegelungs- und Drehermöglichungselement (16) derart angeordnet ist, dass es von einem Benutzer manuell aktiviert wird, wodurch direkt oder indirekt bewirkt wird, dass es verschoben wird, nachdem der zweite Flüssigkeitsbehälter mit der zweiten Komponente (14) verbunden worden ist und/oder der erste Flüssigkeitsbehälter (28) mit der ersten Komponente (12) verbunden worden ist, wodurch durch das Verschieben des Verriegelungs- und Drehermöglichungselements (16) der mindestens eine Vorsprung (18b) aus dem mindestens einen Hohlraum (20) gedrückt wird.

8. Anschluss (10) nach einem der vorhergehenden Ansprüche, **dadurch gekennzeichnet, dass** er einen Schnappverschlussmechanismus (17) zum Verbinden der ersten Komponente (12) mit der zweiten Komponente (14) oder dem Verriegelungs- und Drehermöglichungselement (16) umfasst.

9. Anschluss (10) nach einem der vorhergehenden Ansprüche, **dadurch gekennzeichnet, dass** die erste Komponente (12) derart angeordnet ist, dass sie in die zweite Komponente (14) eingeschraubt wird.

10. Anschluss (10) nach einem der Ansprüche 1-8, **dadurch gekennzeichnet, dass** die erste Komponente (12) derart angeordnet ist, dass sie in die zweite Komponente (14) eingeschoben wird.

11. Anschluss (10) nach einem der vorhergehenden Ansprüche, **dadurch gekennzeichnet, dass** die zweite Komponente (14) und/oder die erste Komponente (12) eine Membran umfasst.

12. Anschluss (10) nach einem der vorhergehenden Ansprüche, **dadurch gekennzeichnet, dass** er ein Dichtungsmittel (15) zum Sicherstellen umfasst, dass eine flüssigkeitsdichte leckfreie Verbindung zwischen dem ersten Flüssigkeitsbehälter (28) und dem zweiten Flüssigkeitsbehälter hergestellt wird.

## Revendications

1. Connecteur (10) pour permettre un transfert de fluide entre un premier récipient de fluide (28) et un deuxième récipient de fluide, lequel connecteur (10) comprend un premier composant (12) qui est agencé pour être relié à un premier récipient de fluide (28) et un deuxième composant (14) qui est agencé pour être relié à un deuxième récipient de fluide, moyennant quoi ledit premier composant (12) est agencé pour être relié de manière non rotative audit deuxième composant (14), ou à une partie du deuxième composant (14), et à un premier récipient de fluide (28) avant qu'un deuxième récipient de fluide ne soit relié audit deuxième composant (14) et/ou avant qu'un premier récipient de fluide (28) ne soit relié au premier composant (12), **caractérisé en ce que** ledit connecteur (10) comprend un élément (16) de verrouillage permettant la rotation qui est agencé pour être activé, et **en ce que** ledit élément permettant la rotation (16) est déplacé de manière non rotative et coulissante par rapport au deuxième composant (14) une fois qu'un deuxième récipient de fluide a été relié audit deuxième composant (14), et/ou une fois qu'un premier récipient de fluide (28) a été relié au premier composant (12), laquelle activation de l'élément de verrouillage permettant la rotation (16) permet au premier composant (12) et au deuxième composant (14) de tourner librement l'un par rapport à l'autre.

2. Connecteur (10) selon la revendication 1, **caractérisé en ce que** l'un desdits premier (12) et deuxième (14) composants comprend au moins une saillie (18b) qui est agencée pour se situer dans au moins une cavité correspondante (20) dans l'autre desdits premier et deuxième (14) composants ou dans ledit élément de verrouillage permettant la rotation (16), afin de relier ledit premier composant (12) audit deuxième composant (14) d'une manière non rotative, et moyennant quoi ledit élément de verrouillage permettant la rotation (16) est agencé pour forcer ladite au moins une saillie (18b) en dehors de ladite au moins une cavité correspondante (20), moyennant quoi ledit premier composant (12) et ledit deuxième composant (14) sont autorisés à tourner librement l'un par rapport à l'autre lorsque ladite au moins une saillie (18b) est forcée en dehors de ladite au moins une cavité correspondante (20).

3. Connecteur (10) selon la revendication 2, **caractérisé en ce que** ledit élément de verrouillage permettant la rotation (16) est agencé pour forcer ladite au moins une saillie (18b) en dehors de ladite au moins une cavité correspondante (20) et dans au moins une fente (24), moyennant quoi ledit premier composant (12) et ledit deuxième composant (14) sont autorisés à tourner librement l'un par rapport à l'autre lorsque ladite au moins une saillie (18b) est située dans ladite au moins une fente (24).

4. Connecteur (10) selon l'une des revendications précédentes, **caractérisé en ce que** ledit élément de verrouillage permettant la rotation (16) est agencé pour être automatiquement activé lorsque ledit deuxième récipient de fluide est relié audit deuxième composant (14) et/ou une fois qu'un premier récipient de fluide (28) a été relié au premier composant (12).

5. Connecteur (10) selon la revendication 4, lorsqu'elle dépend de la revendication 2 ou 3, **caractérisé en ce que** ledit élément de verrouillage permettant la rotation (16) est agencé pour être automatiquement activé lorsqu'il est directement ou indirectement déplacé par ledit deuxième récipient de fluide lorsque ledit deuxième récipient de fluide est ensuite relié audit deuxième composant (14), moyennant quoi ledit déplacement dudit élément de verrouillage permettant la rotation (16) force ladite au moins une saillie (18b) en dehors de ladite au moins une cavité (20) et/ou lorsqu'il est directement ou indirectement déplacé par ledit premier récipient de fluide (28) lorsque ledit premier récipient de fluide (28) est ensuite relié audit premier composant (12), moyennant quoi ledit déplacement dudit élément de verrouillage permettant la rotation (16) force ladite au moins une saillie (18b) en dehors de ladite au moins une cavité (20).

6. Connecteur (10) selon l'une des revendications 1 à 3, **caractérisé en ce que** ledit élément de verrouillage permettant la rotation (16) est agencé pour être activé manuellement par un utilisateur après que ledit deuxième récipient de fluide a été relié audit deuxième composant (14) et/ou lorsque ledit premier récipient de fluide (28) a été relié audit premier composant (12).

7. Connecteur (10) selon la revendication 6 lorsqu'elle dépend de la revendication 2 ou 3, **caractérisé en ce que** ledit élément de verrouillage permettant la rotation (16) est agencé pour être activé manuellement par un utilisateur qui l'amène directement ou indirectement à se déplacer après que ledit deuxième récipient de fluide a été relié audit deuxième composant (14) et/ou ledit premier récipient de fluide (28) a été relié audit premier composant (12), moyennant quoi ledit déplacement dudit élément de verrouillage permettant la rotation (16) force ladite au moins une saillie (18b) en dehors de ladite au moins une cavité (20).

8. Connecteur (10) selon l'une des revendications précédentes, **caractérisé en ce qu'**il comprend un mécanisme d'encliquetage (17) pour relier ledit premier composant (12) audit deuxième composant (14) ou audit élément permettant la rotation (16).

9. Connecteur (10) selon l'une des revendications précédentes, **caractérisé en ce que** ledit premier composant (12) est agencé pour être vissé dans ledit deuxième composant (14).

10. Connecteur (10) selon l'une des revendications 1 à 8, **caractérisé en ce que** ledit premier composant (12) est agencé pour coulisser dans ledit deuxième composant (14).

11. Connecteur (10) selon l'une des revendications précédentes, **caractérisé en ce que** ledit deuxième composant (14) et/ou ledit premier composant (12) comprend/comprennent une membrane.

12. Connecteur (10) selon l'une des revendications précédentes, **caractérisé en ce qu'**il comprend des moyens d'étanchéité (15) pour s'assurer qu'une liaison étanche au fluide et sans fuite est réalisée entre le premier récipient de fluide (28) et le deuxième récipient de fluide.
